# EUROPÄISCHE PATENTANMELDUNG

(11) **EP 0 700 684 A2**
(43) Veröffentlichungstag der Anmeldung: **13.03.1996**
(21) Anmeldenummer: 95112570.7
(22) Anmeldetag: 10.08.1995
(51) Int. Cl.: A61K 38/48, A61K 38/37, A61K 38/36

(54) **Zusammensetzungen, enthaltend Prothrombinkomplex-Konzentrat und wenigstens eine weitere Komponente das die Blutgerrinnung fördert, als Gegenmittel für Blut-Anticoagulanzien**

(30) Priorität: 26.08.1994 DE 4430205
(71) Anmelder: BEHRINGWERKE Aktiengesellschaft, D-35001 Marburg (DE)
(72) Erfinder: Römisch, Jürgen, Dr., D-35041 Marburg (DE); Diehl, Karl-Heinz, Dr., D-35418 Buseck-Oppenrod (DE); Stöhr, Hans-Arnold, D-35083 Wetter (DE)

(57) **Zusammenfassung**

Offenbart werden Zusammensetzungen, die als Gegenmittel für Blut-Antikoagulanzien dienen und eine Kombination von verschiedenen die Blutgerinnung fördernden Komponenten aufweisen, wobei in bevorzugter Form eine der Komponenten das Prothrombinkomplexkonzentrats ist.

## Beschreibung

Gegenstand der Erfindung sind Gegenmittel (Antidots) für Blut-Antikoagulanzien.

Die Blutgerinnung ist ein komplexer Prozeß, an dem Plasmaproteine und Zellen beteiligt sind. Die Bildung eines Wundverschlusses wird durch die rasche Adhäsion von Blutplättchen an die geschädigten Oberflächen und durch Proteasen und Kontaktfaktoren eingeleitet, die nacheinander in einer kaskadenartigen Reaktionsabfolge aktiviert werden. Am Ende dieser Vorgänge steht die Erzeugung von Thrombin, das unter anderem die Aktivierung von Fibrinogen zu Fibrin katalysiert.

Proteine wie die Faktoren VIII und V tragen als Akzelleratoren zur Effizienz dieses Systems bei. Neben dem Protein C-System regulieren Inhibitoren der beteiligten Proteasen im wesentlichen die Gerinnungsprozesse dahingehend, daß ein Wundverschluß zustandekommt, jedoch überschießende Reaktionen vermieden werden. Wird dieses fein abgestimmte Gleichgewicht gestört, kann dies Fibrinablagerungen bzw. die Bildung von Thrombosen zur Folge haben. Bekanntermaßen sind resultierende Gefäßverschlüsse die Ursache für viele pathophysiologische Prozesse, wie insbesondere den Herzinfarkt. Diese Problematik bekommt ein besonderes Gewicht bei solchen Patienten, die einer erhöhten Thrombosegefahr ausgesetzt sind, was häufig eine postoperative Komplikation darstellt.

Zur Verhinderung dieser pathophysiologischen Prozesse werden verschiedene Wege beschritten. So werden zur Verhinderung der Thrombusbildung bzw. zur Unterstützung deren Auflösung (Thrombolyse) in der Klinik häufig Antikoagulanzien angewendet, die in unterschiedlicher Art und Weise in das Gerinnungsgeschehen eingreifen. Neben der Reduktion bzw. Verhinderung der Thrombozytenfunktionen (Aggregation, Adhäsion, Aktivierung, Synthese und Ausschüttung von Aktivierungsprodukten) werden Hemmstoffe von beteiligten Gerinnungsproteasen, insbesondere des Thromboplastin/Faktor VIIa-Komplexes, des Faktors Xa und insbesondere des Thrombins (Faktor IIa) untersucht und angewendet.

Ein besonders spezifischer und potenter Inhibitor des Thrombins ist das Hirudin, das ursprünglich aus den Speicheldrüsen des Blutegels Hirudo medicinalis isoliert wurde. Mittlerweile sind verschiedene Varianten, Analoga, Fragmente und Mutanten davon bekannt und werden auf ihre Tauglichkeit untersucht. In seiner natürlich vorkommenden Form ist das Hirudin sulfatiert, jedoch wurde festgestellt, daß die interessante biologische Aktivität auch bei der nicht sulfatierten Form erhalten bleibt. Daher kann das Hirudin bzw. die Hirudin-Derivate in bequemer Weise mit den Mitteln der Gentechnologie hergestellt werden.

Zu den Blut-Antikoagulanzien zählt auch das Heparin, dessen Fragmente und Varianten sowie synthetische Hemmstoffe, die die katalytische Aktivität des Thrombins und anderer Gerinnungsproteasen, z.B. Faktor Xa, inhibieren. Auch diese Komponenten können bei der Prophylaxe und/oder Therapie von thrombotischen bzw. präthrombotischen Komplikationen eingesetzt werden. Die Suche nach spezifischeren und potenteren Antikoagulanzien bzw. Antithrombotika reflektiert die unbefriedigende Situation, daß die klinisch etablierten Substanzen wie Heparine oder Vitamin K-Antagonisten entweder nicht effektiv genug sind oder, daß sie unerwünschte und zum Teil gravierende Nebenwirkungen aufweisen. Hirudin ist der stärkste derzeit bekannte Thrombininhibitor, der auch nicht in die anderen Enzyme der Blutgerinnungskaskade eingreift. Bisher wurden keine wesentlichen Nebeneinflüsse auf die Herzschlagrate, die Atmung, den Blutdruck, die Anzahl der Thrombozyten, den Fibrinogen- oder Hämoglobingehalt beobachtet. Daher handelt es sich bei Hirudin um ein bevorzugtes Blut-Antikoagulationsmittel.

Die Anwendung von Antikoagulanzien bzw. Antithrombotika am Patienten bringt naturgemäß das Risiko einer erhöhten Blutungsneigung mit sich, die in extremen Situationen, z.B. bei unsachgemäßer Anwendung bzw. Dosierung zu unkontrollierten Blutungen führen kann. Um in einer derartigen Situation der unerwünschten Blutungsneigung gegensteuern zu können, ist es bei einigen Substanzen, deren Beschaffenheit (Molekulargewicht, Struktur) es erlaubt, möglich, diese schnell durch Dialyse aus dem Blutplasma des Patienten zu entfernen. Wenn dies aber aufgrund der apparativen Ausstattung oder aus sonstigen Gründen nicht möglich ist, muß ein Gegenmittel, das auch als Antidot bezeichnet wird, zur Verfügung gestellt werden.

Als Gegenmittel sind bereits einige Substanzen bzw. Präparate vorgeschlagen worden. So zeigen die Thrombine und deren blockierte Varianten einen gewissen Hirudin-neutralisierenden Effekt, jedoch ist einerseits die Anwendung von aktivem Thrombin bei Patienten, die mit Blut-Antikoagulanzien behandelt werden, in der Regel kontra-indiziert und andererseits ist die Präparation von inaktivem Thrombin durch Anwendung niedermolekularer, kovalente Bindungen eingehende Inhibitoren aufgrund der Toxizität dieser Substanzen problematisch. Außerdem fehlen auch Erfahrungen, ob diese Komplexe eine möglicherweise antigene Wirkung besitzen. Diese Komplexe sind auch aufgrund ihrer Natur nicht geeignet, niedermolekulare (synthetische) Antithrombine bzw. Antithrombotika zu neutralisieren. Das Meizothrombin, ein Intermediärprodukt, das bei der Aktivierung vom Prothrombin zu Thrombin kurzfristig entsteht, besitzt zwar eine gewisse Potenz zur Antagonisierung, jedoch kann es sich in vivo schnell zu Thrombin umwandeln und ist als solches in der Regel kontraindiziert.

Die Untersuchung des Faktor VII als Hirudin-Antidot in entsprechenden Modellen erbrachte keine befriedigenden Ergebnisse. In der europäischen Patentanmeldung EPA 89.810733.9 wird die Verwendung von Faktor VIII oder Fragmenten des Faktors VIII als Gegenmittel für Blut-Antikoagulanzien beschrieben. Bekannt ist auch, daß solche Substanzen verwendet werden können, die die Konzentration an Faktor VIII im Blut erhöhen, wie beispielsweise das Desmopressin (Mannucci, P.M.; Progress in Haemostasis and Thrombosis, 8 (1986), S. 19-45).

Da aber auch diese Gegenmittel nicht für alle Anwendungsbereiche ausreichen, besteht nach wie vor die Notwendigkeit, effektivere und an Nebenwirkungen ärmere Gegenmittel für Blut-Antikoagulanzien, Antithrombotika bzw. Plättchenantagonisten bereitzustellen.

Unter antikoagulatorischen bzw. antithrombotischen Substanzen werden im Rahmen der vorliegenden Anmeldung Glykosaminoglykane und sulfatierte Polysaccharide, darunter Heparine, im besonderen unfraktioniertes und niedermolekulares (LMW-) Pentosanpolysulfat, Heparansulfat, Dermatansulfat oder Condroitinsulfat sowie Hirudine, deren Fragmente, z. B. Hirugen (J. Biol. Chem. 265 (1990), 13484-13487; EP-A-0 333 356), Analoga oder Mutanten, z. B. Hirulog® (WO 92/13952), gekoppelte Hirudine, z. B. PEG-Hirudin oder Hirutonine, natürliche oder- synthetische Inhibitoren des Thromboplastin/Faktor VIIaKomplexes, des Faktors Xa oder des Thrombins, Komponenten des Protein C-Systems, nämlich aktiviertes Protein C, Protein S, Thrombomodulin, Fibrinaggregations- oder Vernetzungshemmstoffe, Substanzen aus der Vitamin K-Antagonistengruppe, Substanzen, die die Plättchenaktivierung und/oder -aggregation bzw. Plättchenadhäsion beeinflussen, wie insbesondere Fibrinogenrezeptor-Antagonisten, oder die endogene Fibrinolysekapazität zu steigern vermögen, wie insbesondere PAI-1 Inhibitoren oder Synthese- und Sekretionsstimulatoren von Plasminogenaktivator-Inhibitoren und somit indirekt antithrombotisch wirken, verstanden.

Da die genannten gekoppelten Hirudine, z. B. PEG-Hirudin, aus dem Organismus nur schwer entfernbar sind, ist die Bereitstellung eines geeigneten Antidots für gekoppelte Hirudine besonders wichtig.

Gegenstand der vorliegenden Erfindung sind daher Zusammensetzungen, die als Gegenmittel für Blut-Antikoagulanzien dienen, wobei diese Blut-Antikoagulanzien ausgewählt sein können aus der Gruppe umfassend Glykosaminoglykane und sulfatierte Polysaccharide sowie Hirudine, deren Fragmente, Analoga oder Mutanten, natürliche oder synthetische Inhibitoren des Thromboplastins/Faktor VIIa-Komplexes, des Faktors Xa oder des Thrombins, Komponenten des Protein C-Systems, nämlich aktiviertes Protein C, Protein S, Thrombomodulin, Fibrinaggregations- oder Vernetzungshemmstoffe, Substanzen aus der Vitamin K-Antagonistengruppe, Substanzen, die die Plättchenaktivierung und/oder -aggregation bzw. Plättchenadhäsion beeinflussen, wie insbesondere Fibrinogenrezeptor-Antagonisten, oder die endogene Fibrinolysekapazität zu steigern vermögen, wie insbesondere PAI-1 Inhibitoren oder Synthese- und Sekretionsstimulatoren von Plasminogenaktivator-Inhibitoren, die dadurch gekennzeichnet sind, daß sie ein Prothrombinkomplex-Konzentrat und wenigstens eine weitere, die Blutgerinnung fördernde Komponente aufweisen.

Die erfindungsgemäß verwendeten Prothrombinkomplex-Konzentrate werden durch Anreicherung aus den entsprechenden Blutpräparaten gewonnen. Die Prothrombinkomplex-Konzentrate enthalten, insbesondere die Faktoren II, VII, IX und X. Die Prothrombinkomplex-Konzentrate (PPSB) können in der nicht aktivierten Form vorliegen oder in der aktivierten Form, wobei die Aktivierung üblicherweise dadurch erfolgt, daß das Prothrombinkomplex-Konzentrat z. B. mit Thrombin oder Kontaktaktivatoren umgesetzt wird. Die aktivierten Prothrombinkomplex-Konzentrate reagieren daher bedeutend schneller und sind entsprechend schlechter kontrollierbar. Im Rahmen der vorliegenden Erfindung sind aber die nicht aktivierten Prothrombinkomplex-Konzentrate bevorzugt.

In einer bevorzugten Ausführungsform ist die weitere die Blutgerinnung fördernde Komponente der Faktor VIII, wobei in besonders bevorzugter Weise die weitere die Blutgerinnung fördernde Komponente eine Kombination aus Faktor VIII und dem von Willebrand Faktor (vWF) ist.

In anderen ebenfalls bevorzugten Ausführungsformen ist die weitere die Blutgerinnung fördernde Komponente der Faktor V, der Faktor Va, der von Willebrand Faktor, der Faktor XI, der Faktor XII oder der Faktor XIII, insbesondere die Faktoren V und VIII. Gegebenenfalls können auch Mischungen dieser Komponenten verwendet werden.

Diese Komponenten können auch in Form von aktiven Fragmenten oder Varianten vorliegen, die insbesondere gentechnologisch hergestellt werden können. In einer anderen bevorzugten Ausführungsform der vorliegenden Erfindung ist die weitere die Blutgerinnung fördernde Komponente wenigstens ein Lipid oder ein Gemisch aus Lipiden, wobei es sich bevorzugt um negativ geladene Lipide bzw. Lipidgemische handelt.

In einer anderen bevorzugten Ausführungsform der vorliegenden Erfindung ist die weitere die Blutgerinnung fördernde Komponente wenigstens ein Diuretikum.

Besonders bevorzugt sind Zusammensetzungen, die ein Prothrombinkomplex-Konzentrat und wenigstens zwei weitere die Blutgerinnung fördernde Komponenten - wie oben beschrieben umfassen.

Bei den erfindungsgemäßen Zusammensetzungen werden die Prothrombinkomplex-Konzentrate in einer Konzentration von 1 bis 2000 Internationalen Einheiten (IU) pro Kilogramm Körpergewicht des zu behandelnden Patienten eingesetzt, wobei Konzentrationen des Prothrombinkomplex-Konzentrats in einer Menge von 10 bis 1000 IU/kg besonders bevorzugt sind.

Die weitere die Blutgerinnung fördernde Komponente liegt üblicherweise in einer Konzentration von jeweils 1 bis 2000 IU/kg, wobei auch hier Konzentrationen von 10 bis 1000 IU/kg besonders bevorzugt sind.

Das Diuretikum und/oder das Lipid bzw. Lipidgemisch liegt üblicherweise in einer Konzentration von 0,01 bis 100 mg/kg Körpergewicht des Patienten vor, wobei Konzentrationen des Diuretikums und/oder des Lipids bzw. des Lipidgemisches in einer Menge von 0,1 bis 10 mg/kg besonders bevorzugt sind.

In einer weiteren Ausführungsform betrifft die vorliegende Erfindung Zusammensetzungen, die als Gegenmittel für Blut-Antikoagulanzien dienen, wobei die Blut-Antikoagulanzien ausgewählt sein können aus der Gruppe umfassend Glykosaminoglykane und sulfatierte Polysaccharide sowie Hirudine, deren Fragmente, Analoga oder Mutanten, natürliche und synthetische Inhibitoren des Thromboplastins/Faktor VIIaKomplexes, des Faktors Xa oder des Thrombins, Komponenten des Protein C-Systems, nämlich aktiviertes Protein C, Protein S, Thrombomodulin, Fibrinaggregations- oder Vernetzungshemmstoffe, Substanzen aus der Vitamin K-Antagonistengruppe, Substanzen, die die Plättchenaktivierung und/oder -aggregation bzw. Plättchenadhäsion beeinflussen, wie insbesondere Fibrinogenrezeptor-Antagonisten, oder die endogene Fibrinolysekapazität zu steigern vermögen, wie insbesondere PAI-1 Inhibitoren oder Synthese- und Sekretionsstimulatoren von Plasminogenaktivator-Inhibitoren, die dadurch gekennzeichnet sind, daß sie einerseits wenigstens eine Komponente ausgewählt aus der Gruppe Lipid, Lipidgemisch und Diuretikum und andererseits wenigstens eine Komponente ausgewählt aus der Gruppe Prothrombinkomplex-Konzentrat, jeweils einem der Faktoren II, V, Va, VIII, IX, X, XI, XII, XIII, von Willebrand Faktor als Einzelfaktor oder Mischungen dieser Faktoren umfassen.

In einer weiteren Ausführungsform betrifft die vorliegende Erfindung eine Zusammensetzung, die als Gegenmittel für Blut-Antikoagulanzien dient, wobei die Antikoagulanzien ausgewählt sein können aus der Gruppe umfassend Glykosaminoglykane und sulfatierte Polysaccharide sowie Hirudine, deren Fragmente, Analoga oder Mutanten, natürliche und synthetische Inhibitoren des Thromboplastins/Faktor VIIa-Komplexes, des Faktors Xa oder des Thrombins, Komponenten des Protein C-Systems, nämlich aktiviertes Protein C, Protein S, Thrombomodulin, Fibrinaggregations- oder Vernetzungshemmstoffe, Substanzen aus der Vitamin K-Antagonistengruppe, Substanzen, die die Plättchenaktivierung und/oder -aggregation bzw. Plättchenadhäsion beeinflussen, wie insbesondere Fibrinogenrezeptor-Antagonisten, oder die endogene Fibrinolysekapazität zu steigern vermögen, wie insbesondere PAI-1 Inhibitoren oder Synthese- und Sekretionsstimulatoren von Plasminogenaktivator-Inhibitoren, die dadurch gekennzeichnet sind, daß sie jeweils wenigstens eine Komponente ausgewählt aus einer der beiden Gruppen A und B umfaßt, wobei die Gruppe A die Faktoren II, V, Va, VIII und IX umfaßt und die Gruppe B die Faktoren II, V, Va, VII, VIIa, VIII, den von Willebrand Faktor, die Faktoren IX, X, XI, XII und XIII umfaßt, mit der Maßgabe, daß die Komponente oder die Komponenten aus der Gruppe A nicht identisch ist mit der Komponente oder den Komponenten aus der Gruppe B.
Die Erfindung betrifft auch die Verwendung einer der oben beschriebenen Zusammensetzungen als Gegenmittel für Blut-Antikoagulanzien.

Die vorliegende Erfindung wird durch die nachfolgenden Beispiele weiter erläutert.

### Beispiel 1

In diesem in vitro Versuch wurde das Potential von F VIII (Beriate®), einem nichtaktivierten ProthrombinkomplexKonzentrat (PPSB / Heparin-freies Beriplex®) und deren Kombination zur Antagonisierung der gerinnungshemmenden Wirkung durch Hirudin in Standard-Humanplasma (SHP) getestet.

Dazu wurden 100 µl Citrat-SHP nach Zugabe von je 2 µl einer entsprechend konzentrierten Stammlösung auf eine Endkonzentration von 3 bzw. 20 µg/ml Hirudin gebracht. Nach Mischung mit 50 µl einer konzentrierten Lösung enthaltend F VIII und/oder PPSB und Zugabe von 100 µl Pathromtin®/Kaolin-Suspension schloß sich eine Inkubationszeit von 2 Min. für 37°C an. Die Reaktion wurde durch Beimischung von 50 µl einer 50 mM CaCl₂-Lösung gestartet. Die (modifizierte) aktivierte Partielle Thromboplastinzeit (aPTT) wurde nach Schnitger und Gross bestimmt.

Die in der Tabelle 1 aufgelisteten Gerinnungszeiten verdeutlichen, daß die durch 3 µg/ml Hirudin bewirkte Verlängerung der aPTT (gegenüber Kontrolle), durch 2 IU F VIII verkürzt wurde. Die durch PPSB allein bewirkte Verlängerung ist möglicherweise auf einen bisher nicht vollständig geklärten in vitro Effekt zurückzuführen. Als besonders effektiv stellte sich jedoch deren Kombination heraus. Dieser Effekt wurde umso deutlicher, wenn sehr hohe Hirudin-Konzentrationen (20 µg/ml) eingesetzt wurden, durch die das nicht antagonisierte Plasma selbst nach 2000 Sekunden nicht geronn. 1 IU PPSB allein zeigte einen gewissen verkürzenden Effekt, wogegen 2 IU F VIII allein keine Gerinnung auslösen konnte. Dagegen zeigte sich wieder die Kombination F VIII/PPSB als deutlich besser als die Einzelkomponenten.

**Tabelle 1**

| | **aPTT (sec.)** | | | |
|---|---|---|---|---|
| **Hirudin- Konz. (µg/ml)** | **Kontrolle** | **2 IU F VIII / ml** | **1 IU PPSB /ml** | **2 IU F VIII + 1 IU PPSB/ml** |
| 0 | 36,4 | 31,5 | 31,4 | 24,7 |
| 3 | 98,1 | 76,0 | 121,1 | 57,7 |
| 20 | >2000 | >2000 | 313,6 | 92,7 |

Tabelle 2 zeigt verschiedene Kombinationen von F VIII/PPSB-Konzentrationen, die die o.g. Versuchsreihe komplettieren und die Ergebnisse bestätigen (bei Anwendung von 10 µg/ml Hirudin gegenüber Kontrolle).

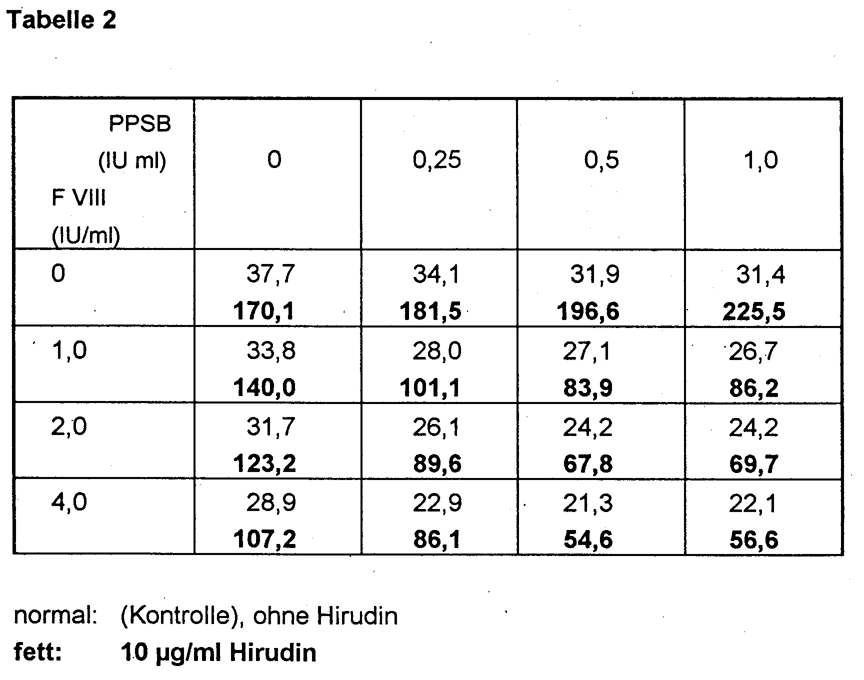

### Beispiel 2

a) Entsprechend Beispiel 1 wurde Ratten Citrat-Plasma, das mit 10 µg/ml Hirudin versetzt wurde, in der aPTT auf antagonisierende Wirkung durch F VIII, PPSB und deren Kombinationen untersucht. Die in der Tabelle 3 demonstrierten Gerinnungszeiten zeigen wiederum, daß die Kombination eine effektivere Normalisierung bewirkt als die Einzelkomponenten. 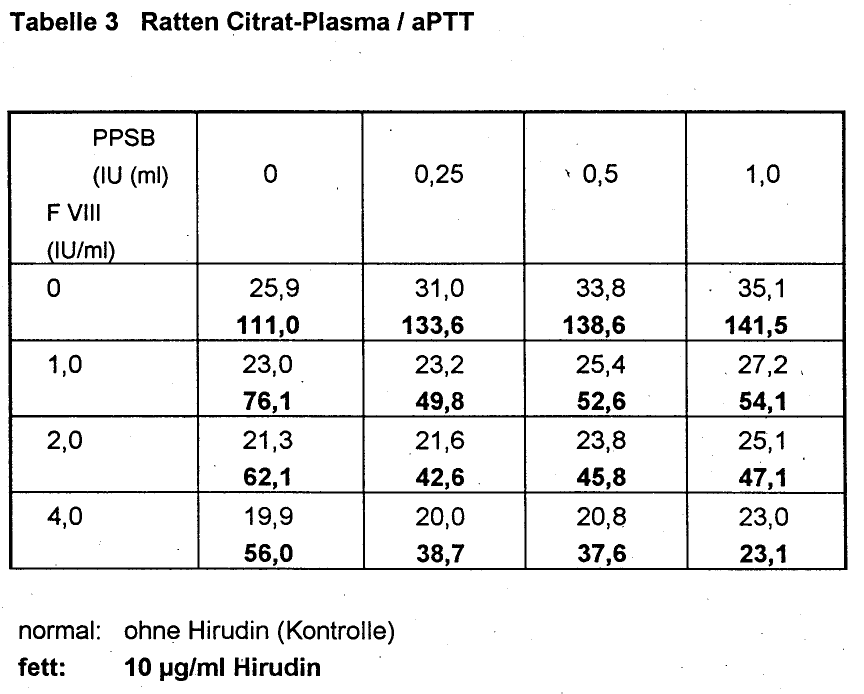
b) Versuche mit Ratten-Vollblut, das im Thrombelastographen (TEG) untersucht wurde, um sich der in vivo-Situation weiter anzunähern, bestätigen die in der aPTT beschriebenen Ergebnisse (Tabelle 4). Der r-Wert (Reaktionszeit) beschreibt prinzipiell die Zeit bis zum Einsetzen der Gerinnung, d.h. die Zeit von der Initiation der Reaktion (Blutabnahme oder Rekalzifizierung) bis zur Verbreiterung der TEG-Kurve um 1 mm. Der k-Wert (Gerinnselbildungszeit) entspricht der Zeit, die vom Endpunkt des r-Wertes bis zur Verbreiterung der Kurve auf 20 mm verstreicht.

Dazu wurden 200 µl Ratten Citrat-Vollblut mit 50 µl einer entsprechend konzentrierten Hirudin-Stammlösung (in Diethylbarbiturat-Puffer, pH 7,6; 1 % Human-Serum-Albumin) und mit 50 µl Antidot (F VIII, PPSB, F VIII+PPSB) gemischt und für 3 Min. bei 37°C vorinkubiert. Die Reaktion wurde durch Zugabe von 50 µl einer 100 mM CaCl₂-Lösung gestartet.

**Tabelle 4**

| **Ratten-Vollblut/TEG** | | | | |
|---|---|---|---|---|
| Hirudin (µg/ml) | Kontrolle | 2 IU F VIII/ml | 1 IU PPSB/ml | 1 IU PPSB 2 IU F VIII/ml |
| 10 | r>40,0 min. | >40,0 min. | 3,7 min. | 2,0 min. |
| | k>40,0min. | >40,0 min. | 2,4 min. | 1,5 min. |

### Beispiel 3

Entsprechend Beispiel 1 wurde die antagonisierende Wirkung der Kombination Faktor V und PPSB getestet. Wieder zeigte sich, daß die Applikation der Kombination den Einzelkomponenten überlegen war. Die Ergebnisse sind in Tabelle 5 dargestellt.

**Tabelle 5**

| | **aPTT (sec.)** | | | |
|---|---|---|---|---|
| **Hirudin- Konz. (µg/ml)** | **Kontrolle** | **2 IU F V / ml** | **1 IU PPSB /ml** | **2 IU F V + 1 IU PPSB/ml** |
| 0 | 36,4 | 35,3 | 31,4 | 28,9 |
| 20 | >2000 | >2000 | 313,6 | 156,3 |

### Beispiel 4

Einer ungewollten überschießenden antikoagulatorischen Reaktion bei Anwendung eines niedermolekularen ThrombinInhibitors, beispielsweise Argatroban (MD 805, (2R,4R)-4methyl-1-{N-{3-Methyl-1,2,3,4-tetrahydro-8-quinolinyl)sulfonyl}-L-argininyl}-2-piperidin-carboxylsäure), kann prinzipiell ebenfalls mit den beschriebenen Komponenten, am effektivsten jedoch mit deren Kombination, begegnet werden. Repräsentativ sind die Effekte auf die aPTT von SHP (Durchführung wie in Beispiel 1 beschrieben) in Tabelle 6 dargestellt.

**Tabelle 6**

| **PPSB (IU/ml)** | **aPTT (sec.)** | | |
|---|---|---|---|
| **F VIII (IU/ml)** | **0** | **0,25** | **0,5** |
| 0 | 36,5 | 32,6 | 31,2 |
| | **217,7** | **216,5** | **228,3** |
| 1,0 | 33,4, | 26,9 | 26,1 |
| | **189,8** | **164,4** | **161,7** |
| 2,0 | 31,1 | 24,2 | 23,8 |
| | **177,2** | **137,4** | **135,2** |
| 4,0 | 28,1 | 21,6 | 20,0 |
| | **164,1,** | **122,4** | **114,8** |
| normal: ohne MD 805, Kontrolle **fett: 10 µg/ml MD 805** | | | |

Ein ähnlicher Effekt ist auch bei Applikation von bivalenten Thrombininhibitoren vom Typ der Hiruloge®, wie z.B. D-Phe-Pro-Arg-Pro-Gly-Gly-Gly-Gly-Asn-Gly-Asp-Phe-Glu-Glu-Ile-Pro-Glu-Glu-Tyr-Leu (Analog), zu erzielen, wie in Tabelle 7 beispielhaft dargestellt.

**Tabelle 7**

| | **aPTT (sec.)** | | | |
|---|---|---|---|---|
| **Analog (µg/ml)** | **Kontrolle** | **2 IU F VIII/ ml** | **1 IU PPSB /ml** | **2 IU F VIII 1 IU PPSB/ml** |
| 3,0 | 97,0 | 77,0 | 107,1 | 66,6 |

### Beispiel 5

Entsprechend Beispiel 1 wurde SHP, das mit 10 µg/ml Hirudin versetzt wurde, in der aPTT auf antagonisierende Wirkung durch F VIII, F IX und deren Kombination untersucht. Die in Tabelle 8 demonstrierten Gerinnungszeiten zeigen wiederum, daß auch F IX eine gewisse antagonisierende Wirkung besitzt, jedoch die Kombination den Einzelkomponenten überlegen ist.

**Tabelle 8**

| **F IX (IU/ml)** | **aPTT (sec.)** | | | |
|---|---|---|---|---|
| **F VIII (IU/ml)** | **0** | **1** | **2** | **4** |
| 0 | 137,5 | 121,7 | 113,0 | 107,3 |
| 2 | 103,3 | 90,6 | 86,2 | 78,3 |
| Hirudin-Konzentration: 10 µg/ml | | | | |

### Beispiel 6

Entsprechend Beispiel 1 wurde SHP, das mit 20 µg/ml Hirudin versetzt wurde, in der aPTT auf antagonisierende Wirkung durch F II, F V, F VII, F VIII, F IX, F X und deren Kombinationen untersucht. Die in Tabelle 9 demonstrierten Gerinnungszeiten zeigen wiederum, daß die Kombinationen den Einzelkomponenten überlegen sind. Am wirkungsvollsten stellte sich die Kombination aller sechs Komponenten heraus.

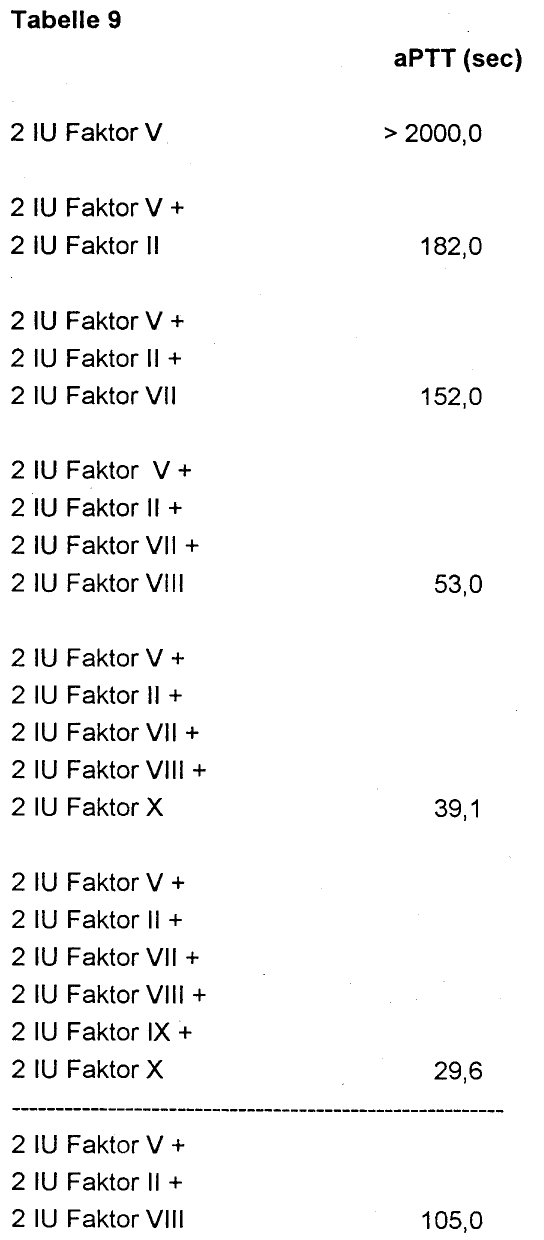

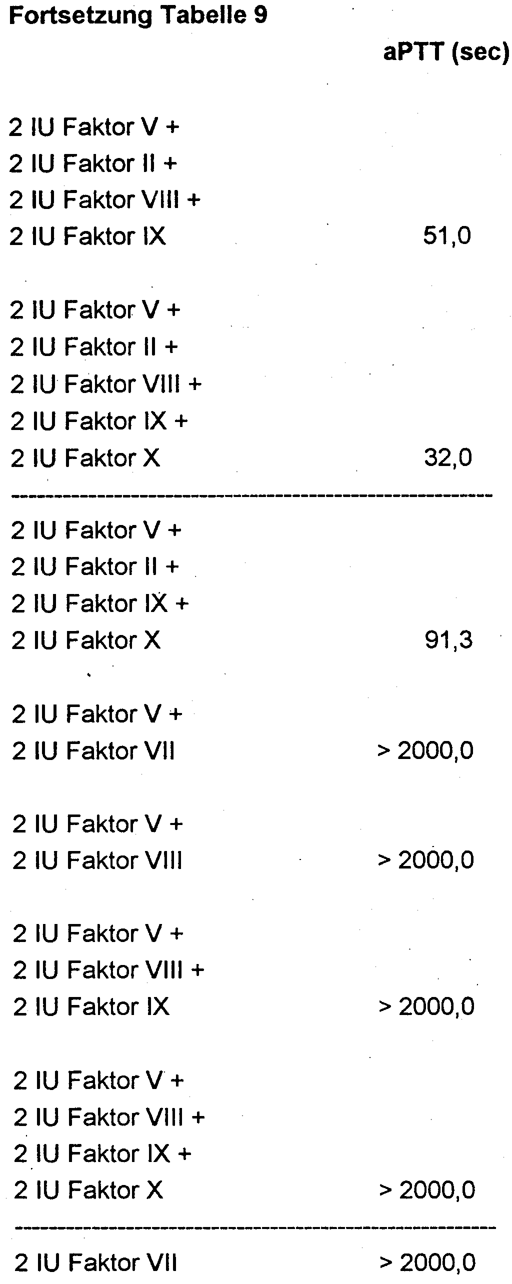

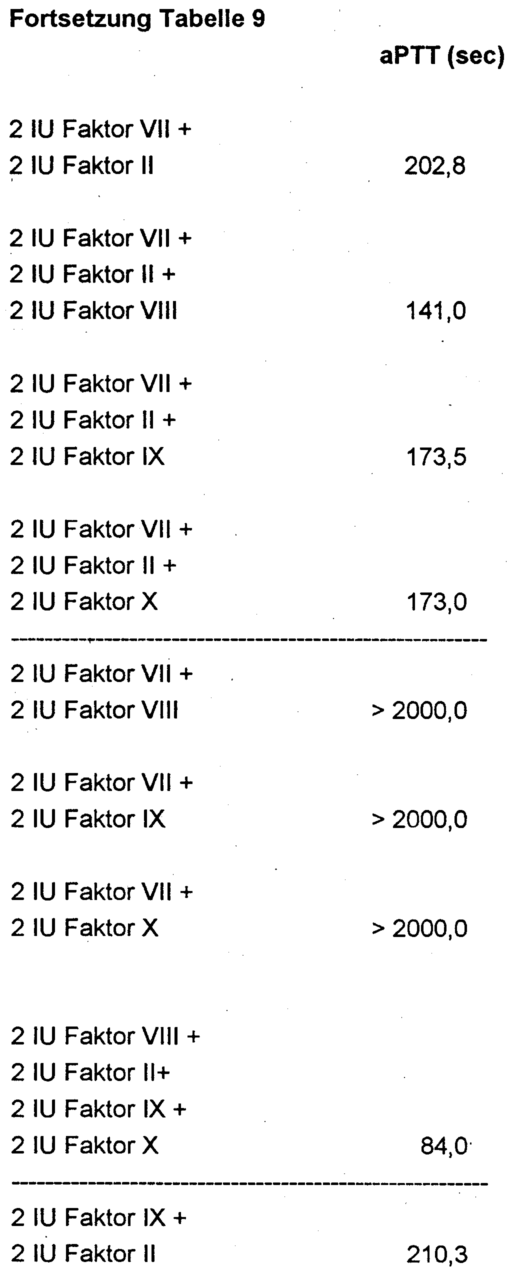

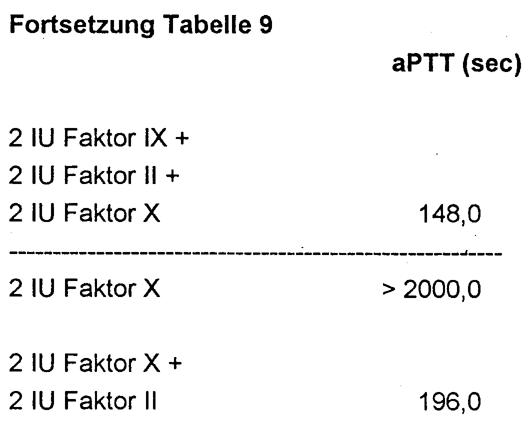

## Patentansprüche

1. Zusammensetzung, die als Gegenmittel für Blut-Antikoagulanzien dient, wobei die Antikoagulanzien ausgewählt sind aus der Gruppe umfassend Glykosaminoglykane und sulfatierte Polysaccharide sowie Hirudine, deren Fragmente, Analoga oder Mutanten, natürliche und synthetische Inhibitoren des Thromboplastins/Faktor VIIa-Komplexes, des Faktors Xa oder des Thrombins, Komponenten des Protein C-Systems, nämlich aktiviertes Protein C, Protein S, Thrombomodulin, Fibrinaggregations- oder Vernetzungshemmstoffe, Substanzen aus der Vitamin K-Antagonistengruppe, Substanzen, die die Plättchenaktivierung und/oder -aggregation bzw. Plättchenadhäsion beeinflussen, wie insbesondere Fibrinogenrezeptor-Antagonisten, oder die endogene Fibrinolysekapazität zu steigern vermögen, wie insbesondere PAI-1 Inhibitoren oder Synthese- und Sekretionsstimulatoren von Plasminogenaktivator-Inhibitoren, dadurch gekennzeichnet, daß sie ein Prothrombinkomplex-Konzentrat und wenigstens eine weitere, die Blutgerinnung fördernde Komponente aufweist.

2. Zusammensetzung nach Anspruch 1, dadurch gekennzeichnet, daß das Prothrombinkomplex-Konzentrat ein nicht aktiviertes Prothrombinkomplex-Konzentrat ist.

3. Zusammensetzung nach Anspruch 1, dadurch gekennzeichnet, daß die weitere die Blutgerinnung fördernde Komponente der Faktor VIII ist.

4. Zusammensetzung nach Anspruch 1, dadurch gekennzeichnet, daß die weitere die Blutgerinnung fördernde Komponente der Faktor VIII und der von Willebrand Faktor (vWF) ist.

5. Zusammensetzung nach Anspruch 1, dadurch gekennzeichnet, daß die weitere die Blutgerinnung fördernde Komponente der Faktor V ist.

6. Zusammensetzung nach Anspruch 1, dadurch gekennzeichnet daß die weitere die Blutgerinnung fördernde Komponente der Faktor Va ist.

7. Zusammensetzung nach Anspruch 1, dadurch gekennzeichnet, daß die weitere die Blutgerinnung fördernde Komponente der von Willebrand Faktor ist.

8. Zusammensetzung nach Anspruch 1, dadurch gekennzeichnet, daß die weitere die Blutgerinnung fördernde Komponente der Faktor XI ist.

9. Zusammensetzung nach Anspruch 1, dadurch gekennzeichnet, daß die weitere die Blutgerinnung fördernde Komponente der Faktor XII ist.

10. Zusammensetzung nach Anspruch 1, dadurch gekennzeichnet, daß die weitere die Blutgerinnung fördernde Komponente der Faktor XIII ist.

11. Zusammensetzung nach Anspruch 1, dadurch gekennzeichnet, daß die weitere die Blutgerinnung fördernde Komponente wenigstens ein Lipid oder ein Gemisch aus Lipiden ist.

12. Zusammensetzung nach Anspruch 11, dadurch gekennzeichnet, daß es sich um negativ geladene Lipide bzw. Lipidgemische handelt.

13. Zusammensetzung nach Anspruch 1, dadurch gekennzeichnet, daß die weitere die Blutgerinnung fördernde Komponente wenigstens ein Diuretikum ist.

14. Zusammensetzung nach Anspruch 1, dadurch gekennzeichnet, daß sie ein Prothrombinkomplexkonzentrat und wenigstens zwei weitere die Blutgerinnung fördernde Komponenten gemäß einem der Ansprüche 3 bis 13 umfaßt.

15. Zusammensetzung nach Anspruch 1, dadurch gekennzeichnet, daß das Prothrombinkomplex-Konzentrat in einer Konzentration von 1 bis 2000 Internationalen Einheiten (IU) pro Kilogramm Körpergewicht des zu behandelnden Patienten vorhanden ist.

16. Zusammensetzung nach Anspruch 15, dadurch gekennzeichnet, daß das Prothrombinkomplex-Konzentrat in einer Menge von 10 bis 1000 IU/kg vorliegt.

17. Zusammensetzung nach Anspruch 1, dadurch gekennzeichnet, daß die weitere die Blutgerinnung fördernde Komponente in einer Konzentration von 1 bis 2000 IU/kg vorliegt.

18. Zusammensetzung nach Anspruch 17, dadurch gekennzeichnet, daß die weitere die Blutgerinnung fördernde Komponente in einer Menge von 10 bis 1000 IU/kg vorliegt.

19. Zusammensetzung nach Anspruch 11, dadurch gekennzeichnet, daß das das Lipid oder Lipidgemisch in einer Konzentration von 0,01 bis 100 mg/kg Körpergewicht des zu behandelnden Patienten vorliegt.

20. Zusammensetzung nach Anspruch 13, dadurch gekennzeichnet, daß das Diuretikum in einer Menge von 0,01 bis 100 mg/kg Körpergewicht des zu behandelnden Patienten vorliegt.

21. Zusammensetzung nach Anspruch 19, dadurch gekennzeichnet, daß das Lipid oder Lipidgemisch in einer Menge von 0,1 bis - 10 mg/kg Körpergewicht des zu behandelnden Patienten vorliegt.

22. Zusammensetzung nach Anspruch 20, dadurch gekennzeichnet, daß das Diuretikum in einer Menge von 0,1 bis 10 mg/kg Körpergewicht des zu behandelnden Patienten vorliegt.

23. Zusammensetzung, die als Gegenmittel für Blutantikoagulanzien dient, wobei die Antikoagulanzien ausgewählt sind aus der Gruppe umfassend Glykosaminoglykane und sulfatierte Polysaccharide sowie Hirudine, deren Fragmente, Analoga oder Mutanten, natürliche und synthetische Inhibitoren des Thromboplastins/Faktor VIIaKomplexes, des Faktors Xa oder des Thrombins, Komponenten des Protein C-Systems, nämlich aktiviertes Protein C, Protein S, Thrombomodulin, Fibrinaggregations- oder Vernetzungshemmstoffe, Substanzen aus der Vitamin K-Antagonistengruppe, Substanzen, die die Plättchenaktivierung und/oder -aggregation bzw. Plättchenadhäsion beeinflussen, wie insbesondere Fibrinogenrezeptor-Antagonisten, oder die endogene Fibrinolysekapazität zu steigern vermögen, wie insbesondere PAI-1 Inhibitoren oder Synthese- und Sekretionsstimulatoren von Plasminogenaktivator-Inhibitoren, dadurch gekennzeichnet, daß sie einerseits wenigstens eine Komponente ausgewählt aus der Gruppe Lipid, Lipidgemisch und Diuretikum und andererseits wenigstens eine Komponente ausgewählt aus der Gruppe Prothrombinkomplex-Konzentrat, jeweils einem der Faktoren II, V, Va, VIII, IX, X, XI, XII, XIII, von Willebrand Faktor als Einzelfaktor oder Mischungen dieser Faktoren umfaßt.

24. Zusammensetzung, die als Gegenmittel für Blut-Antikoagulanzien dient, wobei die Antikoagulanzien ausgewählt sein können aus der Gruppe umfassend Glykosaminoglykane und sulfatierte Polysaccharide sowie Hirudine, deren Fragmente, Analoga oder Mutanten, natürliche und synthetische Inhibitoren des Thromboplastins/Faktor VIIa-Komplexes, des Faktors Xa oder des Thrombins, Komponenten des Protein C-Systems, nämlich aktiviertes Protein C, Protein S, Thrombomodulin, Fibrinaggregations- oder Vernetzungshemmstoffe, Substanzen aus der Vitamin K-Antagonistengruppe, Substanzen, die die Plättchenaktivierung und/oder -aggregation bzw. Plättchenadhäsion beeinflussen, wie insbesondere Fibrinogenrezeptor-Antagonisten, oder die endogene Fibrinolysekapazität zu steigern vermögen, wie insbesondere PAI-1 Inhibitoren oder Synthese- und Sekretionsstimulatoren von Plasminogenaktivator-Inhibitoren, dadurch gekennzeichnet, daß sie jeweils wenigstens eine Komponente ausgewählt aus einer der beiden Gruppen A und B umfaßt, wobei die Gruppe A die Faktoren II, V, Va, VIII und IX umfaßt und die Gruppe B die Faktoren II, V, Va, VII, VIIa, VIII, den von Willebrand Faktor, die Faktoren IX, X, XI, XII und XIII umfaßt, mit der Maßgabe, daß die Komponente oder die Komponenten aus der Gruppe A nicht identisch ist mit der Komponente oder den Komponenten aus der Gruppe B.

25. Verwendung einer Zusammensetzung nach Anspruch 1 zur Herstellung eines Arzneimittels als Gegenmittel für Blut-Antikoagulanzien.
